# FASCICULE DE BREVET EUROPEEN

(11) **EP 4 069 130 B1**
(45) Date de publication et mention de la délivrance du brevet: **18.06.2025**
(21) Numéro de dépôt: 20851323.4
(22) Date de dépôt: 21.12.2020
(51) Int. Cl.: A61B 34/20, A61B 90/00, A61B 34/00

(54) **SYSTEME DE GUIDAGE EN REALITE AUGMENTEE D'UNE OPERATION CHIRURGICALE D'UNE PARTIE D'ARTICULATION D'UN OS**
FÜHRUNGSSYSTEM DER ERWEITERTEN REALITÄT ZUR FÜHRUNG CHIRURGISCHER EINGRIFFE AN EINEM GELENKABSCHNITT EINES KNOCHENS
AUGMENTED REALITY GUIDANCE SYSTEM FOR GUIDING SURGICAL OPERATIONS ON AN ARTICULATING PORTION OF A BONE

(30) Priorité: 20.02.2020 FR 2001685
(43) Date de publication de la demande: 12.10.2022
(73) Titulaire: One Ortho, 69230 Saint-Genis-Laval (FR)
(72) Inventeur: GONZALEZ, Jean-François, 83000 TOULON (FR); COULET, Bertrand, 34980 SAINT-GELY-DU-FESC (FR); ALEPEE, Christophe, 69003 LYON (FR); HOLZER, Nicolas, CH1212 GRAND LANCY (CH)
(74) Mandataire: Cabinet Laurent & Charras
(86) Numéro de dépôt international: PCT/FR2020/052581
(87) Numéro de publication internationale: WO 2021/165587

(56) Documents cités:
- WO-A1-2011/020505
- WO-A1-2019/141704
- US-A1- 2008 319 448
- US-A1- 2018 049 622

## Description

### Domaine technique

La présente invention se rapporte au secteur technique de la chirurgie orthopédique, et concerne plus particulièrement un système de guidage en réalité augmentée d'une opération chirurgicale d'une partie d'articulation d'un os.

L'invention trouve une application pour assister la mise en place de tout type d'implant d'articulation orthopédique telles que prothèses du genou, d'épaule, de hanche et, plus généralement, tout type d'implant orthopédique nécessitant de réaliser des coupes ou perçage au niveau de la zone osseuse anatomique devant être prothésée.

### Art antérieur

Il est parfaitement connu pour un homme du métier d'utiliser différents dispositifs, communément appelés ancillaires de pose pour la mise en place de différents implants orthopédiques. Par exemple, les dispositifs peuvent être constitués par des blocs de guidage tibial, des blocs de guidage fémoral ... Ces blocs intègrent des fentes et orifices dans leur épaisseur pour le passage et le guidage d'instruments à lame de coupe ou foret de perçage, lesquels fentes ou orifices sont convenablement orientés et positionnés en fonction des coupes osseuses ou perçage qu'il convient de réaliser pour la pose de l'implant.

Il est également connu que ces blocs de guidage comprennent au moins une face d'appui sur la partie d'articulation, conçue sur mesure à partir d'un modèle virtuel préalablement établi.

De cette manière, pendant l'intervention, le chirurgien positionne le guide sur-mesure sur l'os, lequel guide doit théoriquement coopérer selon une unique position et orientation avec la partie de l'os, afin de pouvoir guider de manière optimale les opérations de coupe ou de perçage effectuées par le chirurgien.

Cependant, en pratique, le positionnement précis de ce guide sur-mesure reste délicat.

Le document FR 3 078 624 a déjà proposé de tenter de remédier à cet inconvénient en proposant un système d'assistance en réalité augmentée au positionnement d'une instrumentation chirurgicale spécifique impatient.

Selon ce document le système comprend :
- un bloc de guidage destiné à être fixé sur la partie d'articulation de l'os et comprenant, d'une part, au moins une face d'appui sur l'os, conçue sur mesure à partir d'un modèle virtuel osseux et, d'autre part, un repère visuel tridimensionnel ;
- une caméra destinée à visualiser et à suivre le repère ;
- un système de traitement configuré pour traiter des données du modèle virtuel osseux et en temps réel des données images issues de la caméra pour déterminer la position et l'orientation du bloc de guidage, de sorte à afficher sur l'afficheur assujetti à la caméra, et en superposition du champ de vision d'un utilisateur, une représentation virtuelle tridimensionnelle d'au moins une donnée géométrique relative au modèle osseux, dont la position et l'orientation sont déterminées par celles du bloc de guidage.

En d'autres termes, selon ce document, il est possible de visualiser en superposition sur des images réelles du bloc de guidage, un axe mécanique ou des contours de l'os.

Ceci permet d'assister le chirurgien pendant l'opération pour le positionnement correct du bloc de guidage. En pratique, le chirurgien se sert des représentations virtuelles de l'axe mécanique de l'os et des contours, par exemple de l'épiphyse, pour positionner correctement le bloc de guidage, en faisant correspondre et en superposant lesdites représentations virtuelles sur l'os réel.

Cette technique est intéressante, mais repose sur la capacité du chirurgien à faire coïncider les informations virtuelles aux informations réelles, ce qui peut entraîner un risque d'erreur.

En effet, lors de l'opération chirurgicale d'une articulation osseuse, seule ladite articulation est visible par le chirurgien. Les autres parties du membre comprenant l'articulation sont masquées par le champ opératoire, et l'articulation elle-même est tachée de sang, et éventuellement couverte de graisse, ce qui complique l'opération.

Par ailleurs, et d'une manière importante, lorsque le modèle osseux virtuel est obtenu par des techniques du type scanner par exemple, ces techniques ne prennent pas en compte le cartilage de l'os. L'I.R.M. permet de prendre en compte ce cartilage, mais cette technique est contraignante pour le patient, onéreuse, et possède des délais d'attente relativement longs.

De cette manière, le modèle virtuel osseux sur la base duquel est conçu sur-mesure le guide est erroné. Le bloc de guidage a été conçu sur meure pour s'adapter sur un os qui ne comprend pas de cartilage.

Il s'ensuit que lorsque le chirurgien va tenter de positionner le bloc de guidage sur la partie d'articulation à opérer, le cartilage va créer une surépaisseur qui va décaler d'autant les plans de coupe et axes de perçage planifiés et positionnés par le bloc de guidage.

Ainsi, outre la difficulté à trouver le bon positionnement du guide sur-mesure, qui ne s'adapte donc plus de manière très précise à l'os, il en résulte aussi des imprécisions quant au positionnement des différents plans de coupe et axes perçage.

Pour remédier à ce problème, le chirurgien peut décider de retirer le cartilage, mais cette solution n'est pas optimale puisque le retrait du cartilage ne peut être parfaitement maîtrisé.

Une autre solution est de réaliser un guide sur-mesure qui vient en appui sur des zones osseuses où il n'y a pas de cartilage. Cependant, cette technique présente l'inconvénient de fournir des guides très encombrants, et par conséquent une opération plus invasive et avec un risque infectieux accru.

Il est également connu le document WO2019/141704 qui décrit un système de guidage en réalité augmentée d'une opération chirurgicale d'une partie d'articulation d'un os. Cependant, ce document reste muet quant aux difficultés que représente la présence de cartilage pour positionner correctement les éléments de suivi. WO2019/141704 décrit la présence de deux éléments de suivi, fixés à l'os. WO2019/141704 implique donc de faire une incision supplémentaire et de prévoir un ancrage supplémentaire pour fixer le deuxième élément de suivi, ce qui présente des inconvénients pour le patient.

### Exposé de l'invention

L'un des buts de l'invention est de fournir un système de guidage des diverses opérations de coupe ou de perçage d'une articulation osseuse, dont la précision est optimale, tout en restant simple, rapide à mettre en œuvre, avec un risque infectieux limité.

A cet effet, il a été mis au point un système de guidage en réalité augmentée d'une opération chirurgicale d'une partie d'articulation d'un os, le système comprenant :
- un élément de suivi destiné à être fixé sur la partie d'articulation de l'os, l'élément de suivi comprenant, d'une part, au moins une face d'appui sur la partie d'articulation conçue sur mesure à partir d'un modèle virtuel de la partie d'articulation de l'os et, d'autre part, un repère visuel tridimensionnel ;
- une caméra destinée à visualiser et à suivre le repère ;
- un système de traitement configuré pour traiter des données du modèle virtuel osseux et en temps réel des données images issues de la caméra pour déterminer la position et l'orientation de l'élément de suivi, de sorte à afficher sur un afficheur assujetti à la caméra, et en superposition du champ de vision d'un utilisateur, une représentation virtuelle tridimensionnelle d'au moins une donnée géométrique relative au modèle osseux, dont la position et l'orientation sont déterminées par la position et l'orientation de l'élément de suivi.

Selon l'invention, le système de guidage comprend un palpeur aussi équipé d'un repère visuel tridimensionnel et destiné à palper au moins trois zones d'une partie de l'os, hors zone cartilagineuse. Le repère du palpeur est aussi destiné à être suivi par la caméra, et le système de traitement étant en outre configuré pour déterminer la position et l'orientation du palpeur par rapport à la position et l'orientation de l'élément de suivi, de sorte à corriger sur l'afficheur et après palpation, la position et l'orientation de la représentation virtuelle tridimensionnelle de la donnée géométrique relative au modèle osseux par rapport à l'élément de suivi.

De cette manière l'invention permet de positionner un élément de suivi sur la partie d'articulation de l'os, dont la position est connue et repérée avec une précision optimale.

En effet, l'invention ne sert pas à positionner nécessairement directement un bloc de guidage, mais simplement un élément de suivi qui porte un repère et dont on connaît précisément la position par rapport au modèle virtuel osseux.

Ainsi pendant l'intervention, le chirurgien place l'élément de suivi sur l'os, avec l'imprécision liée au cartilage. Le chirurgien se saisit ensuite du palpeur équipé d'un repère, et vient palper des zones, par exemple au moins trois zones de l'os, hors zone cartilagineuse. Cette opération va permettre, en effectuant la correspondance des zones palpées, et celles du modèle virtuel osseux, de recaler précisément la position et l'orientation de la représentation virtuelle de la donnée géométrique relative au modèle virtuel par rapport à la position réelle de l'os.

La donnée géométrique relative au modèle osseux représentée virtuellement et affichée sur l'afficheur peut être de tout type, telle qu'une surface géométrique ou des contours du modèle osseux, un axe mécanique, des plans de coupe ou axes de perçage planifiés, etc.

La technique de palpation, également connu sous l'expression anglaise « *bone morphing »* s'effectue ici seulement sur trois zones, et donc sans le caractère trop invasif, où le risque infectieux connu pour cette technique.

En effet, contrairement à la technique *du « bone morphing »* de l'art antérieur, il n'est pas nécessaire de fixer des broches sur l'os concerné avec des incisions qui ne sont pas justifiées par l'acte chirurgical. Le risque infection n'est donc pas augmenté, et le temps d'intervention n'est pas allongé.

La présente invention permet donc de fixer un repère sur la partie d'articulation, dont on connaît précisément la position, laquelle position a été recalée spécifiquement par rapport à la position réelle de l'os.

Ceci permet d'assister l'utilisation d'autres objets, tels que des blocs de guidage ou des instruments, eux même repérés, dont on connaitra parfaitement leur position relative par rapport à l'élément de suivi et donc par rapport à la représentation virtuelle affichée de la donnée géométrique relative au modèle osseux qui, elle-même sera parfaitement positionnée par rapport à l'os réel.

A cet effet, le système comprend par exemple un instrument de coupe ou de perçage équipé d'un repère visuel tridimensionnel, le repère de l'instrument est aussi destiné à être suivi par la caméra, et le système de traitement étant en outre configuré pour déterminer la position et l'orientation de l'instrument par rapport à la position et l'orientation de l'élément de suivi, de sorte à afficher sur l'afficheur la position et l'orientation d'un plan de coupe ou axe de perçage lié à l'instrument par rapport à l'élément de suivi.

Plusieurs modes de réalisation de l'afficheur peuvent être envisagés sans sortir du cadre de l'invention.

Par exemple, l'afficheur peut être un écran affichant les images issues de la caméra, ou bien peut se présenter sous la forme d'au moins un verre de lunettes, lesquelles lunettes portent éventuellement la caméra, et destinées à être portées par l'utilisateur, l'afficheur n'affichant que les représentations virtuelles.

Avantageusement, la donnée géométrique relative au modèle osseux correspond à la surface externe dudit modèle virtuel osseux, le système de traitement est en outre configuré pour, après correction, traiter les données du modèle virtuel osseux afin d'augmenter ses dimensions au niveau de la surface d'appui de l'élément de suivi pour que l'afficheur représente la face d'appui de l'élément de suivi en contact avec la surface externe du modèle virtuel osseux.

Selon une forme de réalisation, l'élément de suivi comprend des moyens de fixation d'un bloc de guidage, ou bien comprend au moins un orifice de guidage d'une broche de fixation à l'os, et un bloc de guidage présentant au moins un orifice de passage de la broche, le bloc de guidage étant destiné à se fixer à l'os dans une position et une orientation déterminées et guidées par la broche. Par bloc de guidage on entend un bloc qui comprend au moins une fente ou au moins un orifice pour former au moins un guide de coupe ou de perçage.

Avantageusement, le bloc de guidage comprend au moins une fente et/ou au moins un orifice de guidage dont la position et/ou l'orientation sont réglables par rapport au corps du bloc de guidage.

De préférence, et afin de faciliter l'opération chirurgicale, le repère visuel est fixé sur l'élément de suivi de manière amovible ou sécable. Ainsi, lorsque l'élément de suivi a permis de positionner correctement un bloc de guidage, ou directement des instruments de coupe ou de perçage, le repère de l'élément de suivi peut être cassé ou retiré pour ne pas gêner les opérations de coupe ou de perçage en tant que telles.

### Description des figures

D'autres caractéristiques et avantages de l'invention ressortiront clairement de la description qui en est réalisée ci-après, à titre indicatif et nullement limitatif, en référence aux figures annexées dans lesquelles :
[Fig. 1] la figure 1 est une représentation schématique illustrant en perspective un élément de suivi du système selon l'invention, positionné sur une partie d'articulation d'un fémur.
[Fig. 2] la figure 2 est une représentation schématique illustrant l'afficheur, le système de traitement et la caméra du système de guidage en réalité augmentée de l'invention.
[Fig. 3] la figure 3 est une représentation schématique illustrant en perspective un élément de suivi du système selon l'invention, positionné sur une partie d'articulation d'un fémur côté hanche.
[Fig. 4] la figure 4 est une représentation schématique illustrant en perspective un élément de suivi du système selon l'invention, positionné sur une partie d'articulation d'un humérus côté épaule.
[Fig. 5] la figure 5 est une représentation schématique illustrant en perspective un élément de suivi du système selon l'invention, positionné sur une partie d'articulation d'une omoplate.
[Fig. 6] la figure 6 est une représentation schématique illustrant en perspective un élément de suivi du système selon l'invention, positionné sur une partie d'articulation d'un tibia côté genou.
[Fig. 7] la figure 7 est une représentation schématique illustrant en perspective un bloc de guidage fixé sur un tibia et comprenant une fente de guidage dont la position et/ou l'orientation sont réglables par rapport au bloc de guidage.
[Fig. 8] la figure 8 est une représentation schématique illustrant en perspective un palpeur équipé d'un deuxième repère visuel tridimensionnel.
[Fig. 9] la figure 9 est une représentation schématique illustrant en perspective un instrument, tel qu'une perceuse, équipé d'un repère visuel tridimensionnel.

### Description détaillée de l'invention

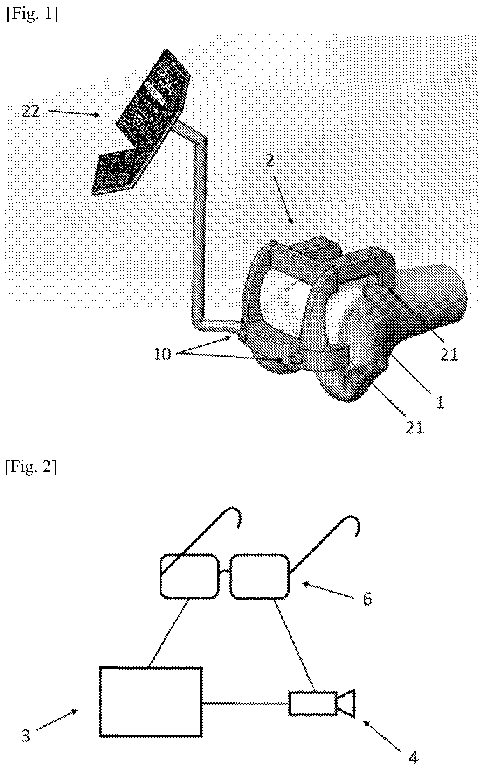

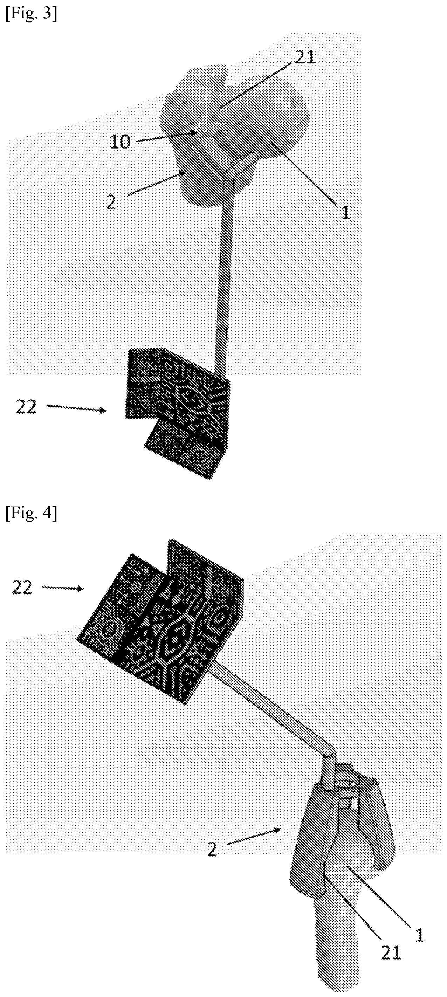

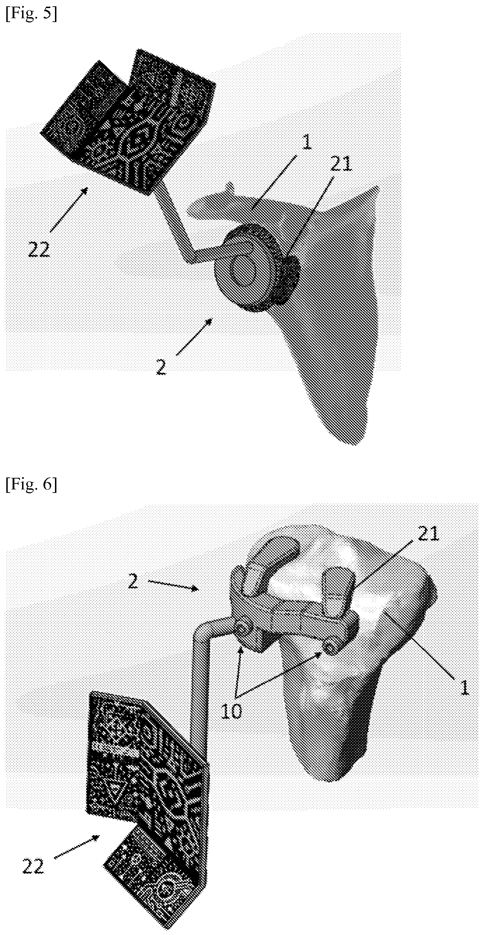

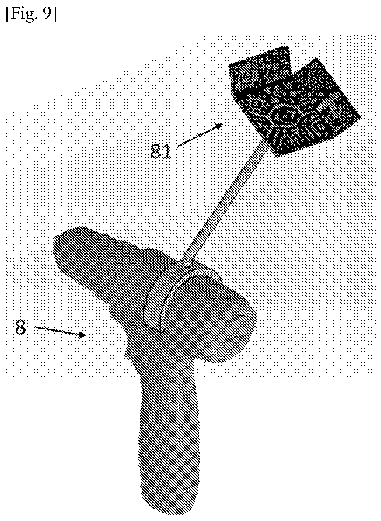

En référence aux figures 1 à 9, l'invention concerne un système de guidage en réalité augmentée d'une opération chirurgicale d'une partie d'articulation (1) d'un os.

L'invention ne se limite pas à une articulation en particulier, et permet de guider la mise en place, c'est-à-dire la réalisation de coupe osseuse ou perçage, de tout type d'implant d'articulation orthopédique, tel que prothèse de genou, d'épaules, de hanches, etc.

D'une manière connue, le système de guidage comprend un élément de suivi (2) destiné à être fixé sur une partie d'articulation (1) d'un os. L'élément de suivi (2) comprend au moins une face d'appui (21) sur la partie d'articulation, conçue sur-mesure à partir d'un modèle virtuel osseux.

Ceci permet de pouvoir créer une relation unique entre le modèle virtuel osseux et l'élément de suivi (2) de sorte que, si l'on connaît la position et l'orientation dans l'espace de l'élément de suivi (2), on connaît donc de manière très précise la position et l'orientation dans l'espace du modèle virtuel osseux.

Afin de pouvoir connaître la position et l'orientation dans l'espace de l'élément de suivi (2), celui-ci comprend, et de manière connue, un repère visuel (22) tridimensionnel intégrant des informations relatives à la position et l'orientation de l'élément de suivi (2), aptes à être traitées par un système de traitement (3), notamment informatique.

Toujours de manière connue, le système de réalité augmentée comprend une caméra (4) destinée à visualiser et à suivre le repère visuel tridimensionnel (22) de l'élément de suivi (2), et un système de traitement (3) configuré pour traiter des données du modèle virtuel osseux et en temps réel des données images issues de la caméra (4) pour déterminer la position et l'orientation de l'élément de suivi (2).

D'une manière connue pour l'homme du métier, la caméra (4) est assujettie, notamment en termes de référentiel, à un afficheur (6) permettant d'afficher en superposition du champ de vision d'un utilisateur des informations transmises par le système de traitement (3).

L'afficheur (6) se présente par exemple sous la forme d'un écran permettant de visualiser directement les images issues de la caméra (4) et de superposer sur celles-ci les informations transmises par le système de traitement (3), ou bien se présente sous la forme de lunettes de réalité augmentée, c'est-à-dire de lunettes comprenant au moins un verre formant l'afficheur (6) et à travers lequel l'utilisateur peut observer une scène, et en superposition de laquelle l'afficheur (6) affiche les informations.

Dans cette dernière forme de réalisation, et d'une manière connue, les lunettes de réalité augmentée peuvent également porter la caméra (4).

De cette manière, lorsque le système de traitement (3) détecte que la caméra (4) visualise le repère visuel tridimensionnel (22) de l'élément de suivi (2), le système de traitement (3) affiche sur l'afficheur (6) une représentation virtuelle tridimensionnelle d'au moins une donnée géométrique relative au modèle osseux positionnée relativement par rapport à l'élément de suivi (2). En d'autres termes, la représentation virtuelle de la donnée géométrique est attachée à l'élément de suivi (2), selon une unique position et orientation qui dépend notamment de celles dudit élément de suivi (2).

La donnée géométrique relative au modèle osseux représentée virtuellement et affichée sur l'afficheur (6) peut être de tout type, telle qu'une surface géométrique ou des contours du modèle osseux, un axe mécanique, des plans de coupe ou axes de perçage planifiés, etc.

Le système de réalité augmentée est bien connu de l'état de la technique et ne sera pas décrit plus en détail. L'invention consiste notamment à corriger l'imprécision de ce système, inhérente à la présence de cartilage ou de matière grasse sur la partie d'articulation sur laquelle doit se fixer l'élément de suivi (2).

Pour ce faire, le système selon l'invention comprend un palpeur (7) aussi équipé d'un repère visuel tridimensionnel (71) portant des informations relatives à la position et l'orientation du palpeur (7) dans l'espace.

Lorsque l'élément de suivi (2) est fixé à l'os réel, l'afficheur (6) va représenter virtuellement la donnée géométrique relative au modèle osseux en superposition de la scène réelle. Cependant, eu égard à la présence de cartilage, il y aura un léger décalage entre l'image réelle de l'os, et la représentation virtuelle. En effet, et en pratique, l'élément de suivi (2) n'est pas directement contact de l'os, mais en contact d'une surépaisseur de cartilage.

Pour corriger ce décalage, le chirurgien va palper, avec le palpeur (7) repéré, au moins trois zones, par exemple trois zones d'environ 2 mm² et non cartilagineuses.

Étant donné que le système de traitement (3) détermine avec précision la position et l'orientation des éléments attachés aux repères (22, 71), la position et l'orientation dans l'espace du palpeur (7) par rapport à la position et à l'orientation de l'élément de suivi (2) est connue.

En théorie, les trois zones que vient palper le palpeur (7) doivent normalement être des zones en correspondance avec des zones équivalentes du modèle virtuel osseux. Le système de traitement (3) est configuré pour rectifier ce décalage et repositionner de manière très précise le modèle virtuel osseux, et notamment la représentation virtuelle de la donnée géométrique, par rapport au repère visuel tridimensionnel (22) de l'élément de suivi (2), et donc par rapport à l'élément de suivi (2) lui-même.

De ce qui précède, ceci permet de connaître très précisément la position de la partie d'articulation de l'os, et de la visualiser en trois dimensions et virtuellement sur l'afficheur (6) en superposition l'os réel. Il est également possible de visualiser l'axe mécanique de l'os, ou bien les plans de coupe ou axes de perçage planifiés.

Pour faciliter ensuite et guider l'opération chirurgicale, le système selon invention comprend également un instrument (8), tel qu'un instrument de coupe ou de perçage, lequel comprend également un repère visuel tridimensionnel (81) portant des informations relatives à la position et l'orientation dudit instrument (8) dans l'espace que le système de traitement (3) est apte à traiter lorsqu'il est visualisé par la caméra (4).

En d'autres termes, il est possible de connaître précisément la position et l'orientation de l'instrument (8) par rapport au repère visuel (22) de l'élément de suivi (2), et donc par rapport à l'os lui-même. Les informations intégrées dans le repère visuel tridimensionnel (81) de l'instrument (8) permettent au système de traitement (3) d'afficher sur l'afficheur (6) et en superposition du champ de vision du chirurgien par exemple, un axe de perçage ou un plan de coupe lié à l'instrument (8) et parfaitement positionné par rapport à l'os réel, sans l'imprécision relative à la surépaisseur de cartilage.

L'élément de suivi (2) peut également comprendre des moyens de fixation d'un bloc de guidage (9), notamment dans une position et une orientation connues et déterminées par rapport à l'élément de suivi (2).

Dans cette configuration, le repère visuel tridimensionnel (22) de l'élément de suivi (2) peut être relié à l'élément de suivi (2) d'une manière amovible ou sécable permettant au chirurgien de le retirer afin de ne pas gêner l'opération de coupe de perçage ultérieure.

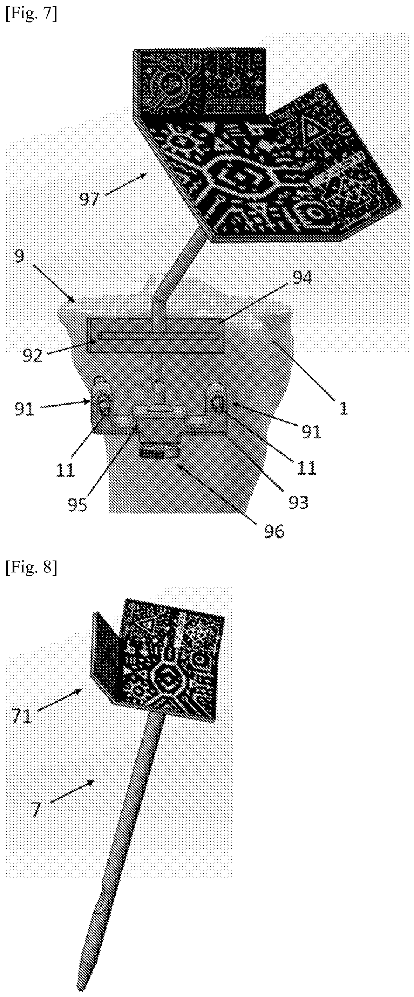

Selon une autre forme de réalisation, l'élément de suivi (2) comprend au moins un, et de préférence deux orifices (10) adaptés pour recevoir deux broches (11) destinées à être fixées dans l'os. L'élément de suivi (2) peut alors être retiré en le faisant coulisser le long des broches (11), et un bloc de guidage (9) comprenant également deux mêmes orifices (91) peut être fixé à l'os en le positionnant par l'intermédiaire des deux broches (11), voir figure 7. Le bloc de guidage (9) comprend une fente (92) et/ou orifice de guidage dont la position et l'orientation sont connues par rapport à l'os.

Afin de pouvoir s'adapter aux besoins du chirurgien, la position et/ou l'orientation des fentes (92) ou orifice de guidage sont réglables par rapport au bloc de guidage (9).

Par exemple, en référence à la figure 7, le bloc de guidage (9) comprend une partie principale (93) destinée à être fixée à l'os par les broches (11) de fixation, et une fente de guidage (92) ménagée dans une partie secondaire (94) du bloc, reliée à la partie principale par une rotule (95). Des moyens de serrage (96), par exemple par vissage, sont assujettis à la rotule (95) pour bloquer son pivotement. La partie secondaire (94) du bloc de guidage (9) comprend un repère visuel tridimensionnel (97) portant des informations relatives à la position et l'orientation de la fente (92) dans l'espace que le système de traitement (3) est apte à traiter lorsqu'il est visualisé par la caméra (4).

En d'autres termes, il est possible de connaître précisément la position et l'orientation de la fente (92) par rapport au repère visuel (22) de l'élément de suivi (2), et donc par rapport à l'os lui-même. Les informations intégrées dans le repère visuel tridimensionnel (97) de la partie secondaire (94) du bloc de guidage (9) permettent au système de traitement (3) d'afficher sur l'afficheur (6) et en superposition du champ de vision du chirurgien par exemple, un plan de coupe ou axe de perçage lié à la partie secondaire (94) du bloc de guidage (9) et parfaitement positionné par rapport à l'os réel, sans l'imprécision relative à la surépaisseur de cartilage.

## Revendications

1. Système de guidage en réalité augmentée d'une opération chirurgicale d'une partie d'articulation (1) d'un os, le système comprenant :
- un élément de suivi (2) destiné à être fixé sur la partie d'articulation de l'os, l'élément de suivi (2) comprenant, d'une part, au moins une face d'appui (21) sur la partie d'articulation conçue sur mesure à partir d'un modèle virtuel de la partie d'articulation de l'os et, d'autre part, un repère visuel tridimensionnel (22) ;
- une caméra (4) destinée à visualiser et à suivre le repère (22) ;
- un système de traitement (3) configuré pour traiter des données du modèle virtuel osseux et en temps réel des données images issues de la caméra (4) pour déterminer la position et l'orientation de l'élément de suivi (2), de sorte à afficher sur un afficheur (6) assujetti à la caméra (4), et en superposition du champ de vision d'un utilisateur, une représentation virtuelle tridimensionnelle d'au moins une donnée géométrique relative au modèle osseux, dont la position et l'orientation sont déterminées par la position et l'orientation de l'élément de suivi (2) ;
- -un palpeur (7) aussi équipé d'un repère visuel tridimensionnel (71) et destiné à palper au moins trois zones d'une partie de l'os hors zone cartilagineuse, le repère (71) du palpeur (7) est aussi destiné à être suivi par la caméra (4),
**caractérisé en ce que**
le système de traitement (3) étant en outre configuré pour déterminer la position et l'orientation du palpeur (7) par rapport à la position et l'orientation de l'élément de suivi (2), de sorte à corriger sur l'afficheur (6) et après palpation, la position et l'orientation de la représentation virtuelle tridimensionnelle de la donnée géométrique relative au modèle osseux par rapport à l'élément de suivi (2).

2. Système de guidage selon la revendication 1, **caractérisé en ce qu'**il comprend un instrument (8) de coupe ou de perçage équipé d'un repère visuel tridimensionnel (81), le repère (81) est destiné à être suivi par la caméra (4), et le système de traitement (3) étant en outre configuré pour déterminer la position et l'orientation de l'instrument (8) par rapport à la position et l'orientation de l'élément de suivi (2), de sorte à afficher sur l'afficheur (6) la position et l'orientation d'un plan de coupe ou axe de perçage lié à l'instrument (8) par rapport à l'élément de suivi (2).

3. Système de guidage selon la revendication 1, **caractérisé en ce que** l'afficheur (6) est un écran affichant les images issues de la caméra (4).

4. Système de guidage selon la revendication 1, **caractérisé en ce que** l'afficheur (6) est au moins un verre de lunettes destinées à être portées par l'utilisateur.

5. Système de guidage selon la revendication 4, **caractérisé en ce que** les lunettes portent la caméra (4).

6. Système de guidage selon la revendication 1, **caractérisé en ce que** la donnée géométrique relative au modèle osseux correspond à la surface externe dudit modèle virtuel osseux, et le système de traitement (3) est en outre configuré pour, après correction, traiter les données du modèle virtuel osseux afin d'augmenter ses dimensions au niveau de la surface d'appui (21) de l'élément de suivi (2) pour que l'afficheur (6) représente la face d'appui (21) de l'élément de suivi (2) en contact avec la surface externe du modèle virtuel osseux.

7. Système de guidage selon la revendication 1, **caractérisé en ce que** le repère visuel (22) est fixé sur l'élément de suivi (2) de manière amovible ou sécable.

8. Système de guidage selon la revendication 1, **caractérisé en ce que** l'élément de suivi (2) comprend des moyens de fixation d'un bloc de guidage (9).

9. Système de guidage selon la revendication 1, **caractérisé en ce que** l'élément de suivi (2) comprend au moins un orifice (10) de guidage d'une broche (11) de fixation à l'os, et le système comprend un bloc de guidage (9) présentant au moins un orifice (91) de passage de la broche (11), le bloc de guidage (9) étant destiné à se fixer à l'os dans une position et une orientation déterminées et guidées par la broche (11).

10. Système de guidage selon les revendications 8 à 9, **caractérisé en ce que** le bloc de guidage (9) comprend au moins une fente (92) et/ou au moins un orifice de guidage dont la position et/ou l'orientation sont réglables par rapport au bloc de guidage (9).

## Patentansprüche

1. Führungssystem für die Augmented-Reality-gestützte Durchführung einer chirurgischen Operation eines Gelenkbereichs (1) eines Knochens, wobei das System umfasst:
- ein Verfolgungselement (2), das dazu bestimmt ist, am Gelenkbereich des Knochens befestigt zu werden, wobei das Verfolgungselement (2) einerseits mindestens eine auf den Gelenkbereich abgestimmte Auflagefläche (21) umfasst, die aus einem virtuellen Modell des Gelenkbereichs des Knochens maßgefertigt ist, und andererseits eine dreidimensionale visuelle Markierung (22) aufweist;
- eine Kamera (4), die dazu bestimmt ist, die Markierung (22) zu visualisieren und zu verfolgen;
- ein Verarbeitungssystem (3), das dazu konfiguriert ist, Daten des virtuellen Knochenmodells und Echtzeit-Bilddaten von der Kamera (4) zu verarbeiten, um die Position und Orientierung des Verfolgungselements (2) zu bestimmen, sodass auf einer Anzeige (6), die mit der Kamera (4) verbunden ist, und im Sichtfeld eines Benutzers eine dreidimensionale virtuelle Darstellung von mindestens einer geometrischen Daten des Knochenmodells angezeigt wird, deren Position und Orientierung durch die Position und Orientierung des Verfolgungselements (2) bestimmt werden;
- einen Taster (7), der ebenfalls mit einer dreidimensionalen visuellen Markierung (71) ausgestattet ist und dazu bestimmt ist, mindestens drei Bereiche eines Teils des Knochens außerhalb des Knorpelbereichs abzutasten, wobei die Markierung (71) des Tasters (7) ebenfalls von der Kamera (4) verfolgt werden soll, **dadurch gekennzeichnet, dass** das Verarbeitungssystem (3) darüber hinaus dazu konfiguriert ist, die Position und Orientierung des Tasters (7) relativ zur Position und Orientierung des Verfolgungselements (2) zu bestimmen, sodass nach dem Abtasten die Position und Orientierung der dreidimensionalen virtuellen Darstellung der geometrischen Daten des Knochenmodells relativ zum Verfolgungselement (2) auf der Anzeige (6) korrigiert werden.

2. Führungssystem nach Anspruch 1, **dadurch gekennzeichnet, dass** es ein Schneid- oder Bohrinstrument (8) umfasst, das mit einer dreidimensionalen visuellen Markierung (81) ausgestattet ist, wobei die Markierung (81) von der Kamera (4) verfolgt werden soll, und das Verarbeitungssystem (3) darüber hinaus dazu konfiguriert ist, die Position und Orientierung des Instruments (8) relativ zur Position und Orientierung des Verfolgungselements (2) zu bestimmen, sodass die Position und Orientierung einer Schneideebene oder Bohrachse, die mit dem Instrument (8) verbunden ist, relativ zum Verfolgungselement (2) auf der Anzeige (6) angezeigt wird.

3. Führungssystem nach Anspruch 1, **dadurch gekennzeichnet, dass** die Anzeige (6) ein Bildschirm ist, der die Bilder der Kamera (4) anzeigt.

4. Führungssystem nach Anspruch 1, **dadurch gekennzeichnet, dass** die Anzeige (6) mindestens eine Brille ist, die vom Benutzer getragen werden soll.

5. Führungssystem nach Anspruch 4, **dadurch gekennzeichnet, dass** die Brille die Kamera (4) trägt.

6. Führungssystem nach Anspruch 1, **dadurch gekennzeichnet, dass** die geometrische Daten des Knochenmodells der äußeren Oberfläche des virtuellen Knochenmodells entsprechen, und das Verarbeitungssystem (3) darüber hinaus dazu konfiguriert ist, nach der Korrektur die Daten des virtuellen Knochenmodells zu verarbeiten, um dessen Dimensionen auf der Auflagefläche (21) des Verfolgungselements (2) zu vergrößern, sodass die Anzeige (6) die Auflagefläche (21) des Verfolgungselements (2) in Kontakt mit der äußeren Oberfläche des virtuellen Knochenmodells darstellt.

7. Führungssystem nach Anspruch 1, **dadurch gekennzeichnet, dass** die visuelle Markierung (22) am Verfolgungselement (2) abnehmbar oder trennbar befestigt ist.

8. Führungssystem nach Anspruch 1, **dadurch gekennzeichnet, dass** das Verfolgungselement (2) Mittel zur Befestigung eines Führungsblocks (9) umfasst.

9. Führungssystem nach Anspruch 1, **dadurch gekennzeichnet, dass** das Verfolgungselement (2) mindestens eine Führungsoffnung (10) für einen Stift (11) zur Befestigung am Knochen umfasst, und das System einen Führungsblock (9) umfasst, der mindestens eine Öffnung (91) zum Durchführen des Stifts (11) aufweist, wobei der Führungsblock (9) dazu bestimmt ist, in einer durch den Stift (11) bestimmten und geführten Position und Orientierung am Knochen befestigt zu werden.

10. Führungssystem nach den Ansprüchen 8 bis 9, **dadurch gekennzeichnet, dass** der Führungsblock (9) mindestens einen Schlitz (92) und/oder mindestens eine Führungsoffnung umfasst, deren Position und/oder Orientierung relativ zum Führungsblock (9) einstellbar ist.

## Claims

1. A guidance system in augmented reality for a surgical operation of a joint part (1) of a bone, the system comprising:
- a tracking element (2) intended to be fixed on the joint part of the bone, the tracking element (2) comprising, on one hand, at least one support face (21) on the joint part custom-designed from a virtual model of the joint part of the bone and, on the other hand, a three-dimensional visual marker (22);
- a camera (4) intended to visualize and track the marker (22);
- a processing system (3) configured to process data from the virtual bone model and realtime image data from the camera (4) to determine the position and orientation of the tracking element (2), so as to display on a display (6) attached to the camera (4), and superimposed on the user's field of vision, a three-dimensional virtual representation of at least one geometric data related to the bone model, whose position and orientation are determined by the position and orientation of the tracking element (2);
- a probe (7) also equipped with a three-dimensional visual marker (71) and intended to probe at least three areas of a part of the bone outside the cartilage zone, the marker (71) of the probe (7) is also intended to be tracked by the camera (4), **characterized in that** the processing system (3) is further configured to determine the position and orientation of the probe (7) relative to the position and orientation of the tracking element (2), so as to correct on the display (6) and after probing, the position and orientation of the three-dimensional virtual representation of the geometric data related to the bone model relative to the tracking element (2).

2. The guidance system according to claim 1, **characterized in that** it comprises a cutting or drilling instrument (8) equipped with a three-dimensional visual marker (81), the marker (81) is intended to be tracked by the camera (4), and the processing system (3) is further configured to determine the position and orientation of the instrument (8) relative to the position and orientation of the tracking element (2), so as to display on the display (6) the position and orientation of a cutting plane or drilling axis related to the instrument (8) relative to the tracking element (2).

3. The guidance system according to claim 1, **characterized in that** the display (6) is a screen displaying images from the camera (4).

4. The guidance system according to claim 1, **characterized in that** the display (6) is at least one lens of glasses intended to be worn by the user.

5. The guidance system according to claim 4, **characterized in that** the glasses carry the camera (4).

6. The guidance system according to claim 1, **characterized in that** the geometric data related to the bone model corresponds to the external surface of said virtual bone model, and the processing system (3) is further configured to, after correction, process the data of the virtual bone model in order to increase its dimensions at the support face (21) of the tracking element (2) so that the display (6) represents the support face (21) of the tracking element (2) in contact with the external surface of the virtual bone model.

7. The guidance system according to claim 1, **characterized in that** the visual marker (22) is fixed on the tracking element (2) in a removable or breakable manner.

8. The guidance system according to claim 1, **characterized in that** the tracking element (2) comprises means for fixing a guidance block (9).

9. The guidance system according to claim 1, **characterized in that** the tracking element (2) comprises at least one guiding orifice (10) for a pin (11) to be fixed to the bone, and the system comprises a guidance block (9) having at least one orifice (91) for the passage of the pin (11), the guidance block (9) being intended to be fixed to the bone in a determined position and orientation guided by the pin (11).

10. The guidance system according to claims 8 to 9, **characterized in that** the guidance block (9) comprises at least one slot (92) and/or at least one guiding orifice whose position and/or orientation are adjustable relative to the guidance block (9)
